(19) Europäisches Patentamt
European Patent Office
Office européen des brevets

(11) Publication number : **0 632 040 A1**

# (12) EUROPEAN PATENT APPLICATION

(21) Application number : **94304891.8**

(22) Date of filing : **04.07.94**

(51) Int. Cl.[6] : **C07D 487/04,** A61K 31/415, A61K 31/50, C07D 471/04, C07D 519/00, A61K 31/42, A61K 31/425, A61K 31/435, // (C07D487/04, 237:00, 235:00), (C07D471/04, 235:00, 221:00), (C07D519/00, 513:00, 487:00)

(30) Priority : **02.07.93 JP 164891/93**

(43) Date of publication of application :
**04.01.95 Bulletin 95/01**

(84) Designated Contracting States :
**AT BE CH DE DK ES FR GB GR IE IT LI LU NL PT SE**

(71) Applicant : **Takeda Chemical Industries, Ltd.**
**1-1 Doshomachi 4-chome,**
**Chuo-ku**
**Osaka-shi, Osaka 541 (JP)**

(72) Inventor : **Yukimasa, Hidefumi**
**683-3, Kiderahigashiguchi-cho,**
**Nara**
**Nara, 630 (JP)**
Inventor : **Nakao, Masafumi**
**720-74, Oze-cho**
**Ikoma, Nara 630-02 (JP)**

(74) Representative : **Lewin, John Harvey**
**Elkington and Fife**
**Prospect House**
**8 Pembroke Road**
**Sevenoaks, Kent TN13 1XR (GB)**

(54) **Antibacterial imidazole derivatives.**

(57) An imidazole compound of by the formula (I')

$$A'\text{-}(CH_2)_m\text{-}X\text{-}(CH_2)_n\text{-}B \qquad (I')$$

wherein A′ is a condensed imidazolyl group having at least one nitro group on the imidazole ring, X is an oxygen atom or a sulfur atom, B is a 5- or 6-membered heterocyclic group which may be a condensed ring, and m and n are each an integer of 0 to 4, or its salt, an antibacterial agent which comprises an imidazole compound of the formula (I) :

$$A\text{-}(CH_2)m\text{-}X\text{-}(CH_2)n\text{-}B \qquad (I)$$

wherein A is an imidazol-1-yl or condensed imidazolyl group having at least one nitro group on the imidazole ring, X is an oxgen atom or a sulfur atom, B is a 5- or 6- membered heterocyclic group which may be a condensed ring, and m and n are each an integer of 0 to 4, or its pharmaceutically acceptable salt and a carrier or diluent and an antiulcer agent which comprises an imidazole compound of the formula (I) as defined above or its pharmaceutically acceptable salt and a carrier or diluent.

EP 0 632 040 A1

BACKGROUND OF THE INVENTION

1. Field of the Invention

The present invention relates to imidazole compounds and, more particularly, it relates to imidazole compounds exhibiting excellent antibacterial activities - particularly high antibacterial activities against microorganisms belonging to the genus *Helicobacter* such as *Helicobacter pylori* - and also to antibacterial agents and antiulcer agents containing the same.

2. Description of the Prior Art

Antacids, anticholinergic agents, antigastrinic agents, gastrointestinal hormones, antipepsinic agents, histamine $H_2$ receptor antagonists, tissue restoring agents, mucous membrane protectors, microcirculation improving agents, proton pump inhibitors, etc. have been developed as the therapeutic agents for ulcers. Therapy of ulcers has become easy as a result of developments of histamine $H_2$ receptor antagonists and proton pump inhibitors having particularly strong inhibitory effect against an acid secretion among the above-mentioned therapeutic agents for ulcers.

However, those therapeutic agents for ulcers are with a high rate of recurrence of the ulcer after ceasing the administration and that has now being left as a big problem to be solved.

*Helicobacter pylori* is a Gram-negative microaerophilic microorganism belonging to the genus *Helicobacter* and has been suggested to exhibit a possibility of a big cause for the recurrence of gastrisis, duodenal ulcer, gastric ulcer, etc.

Two combination use of a bismuth preparation and an antibiotic or three combination use of a bismuth preparation, metronidazole [see, e.g., U. S. Patent No. 2,944,061] and tetracycline [see, e.g., U. S. Patent No. 2,712,517] or amoxicillin [see, e.g., U. S. Patent No.3,192,198] has been currently used as chemotherapy for various diseases caused by said *Helicobacter pylori*. The above-mentioned metronidazole is an imidazole derivative having an activity against *Helicobacter pylori* and has been used together with antibiotics. Those bismuth preparation, antibiotics and metronidazole are administered by an oral route.

However, the above-mentioned bismuth preparation, antibiotics and metronidazole are to be administered in large doses a day for maintaining the concentrations sufficient for inhibiting the growth of *Helicobacter pylori* at its growing location. Thus, there are many problems such as generation of the adverse reactions such as vomiting and diarrhea.

Various compounds having an activity against *Helicobacter pylori* have been reported already. For example, Japanese Laid-Open Patent Publication No. Hei 5(1993)-117,268, discloses pyridine derivatives having an activity against *Helicobacter pylori*, while European Patent (EP 0,535,528 A1) discloses imidazole derivatives having an activity against *Helicobacter pylori*. However, the condensed imidazole compounds which have nitro group(s) are not described.

Further, some imidazole derivatives are disclosed in GB 1,393,228, WO 9000549, DE 2,455,582, US Patent No. 3,992,397, US Patent No. 3,984,426 and US Patent No. 5,013,743, however, in these references an activity against microorganisms belonging to the genus *Helicobacter* is not disclosed.

SUMMARY OF THE INVENTION

Under such circumstances, the present inventors have carried out various investigations and found the imidazole compounds which have excellent antibacterial activity (particularly against microorganisms belonging to the genus *Helicobacter* represented by *Helicobacter pylori*) and further have antiulcer activity in addition to such an antibacterial activity whereby the present invention has been achieved.

Thus, the present invention provides:

(1) an imidazole compound of the formula (I')

$$A'\text{-}(CH_2)_m\text{-}X\text{-}(CH_2)_n\text{-}B \qquad (I')$$

in which A' is a condensed imidazolyl group having at least one nitro group on the imidazole ring, X is an oxygen atom or a sulfur atom, B is a five- or six-membered heterocyclic group which may be a condensed ring, and m and n are each an integer of 0 to 4,

or its salt;

(2) a compound according to (1) above, in which B is a 5-membered heterocyclic group of the formula (VI):

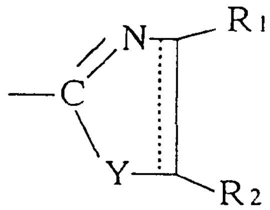

(VI)

wherein $R_1$ and $R_2$ are, the same or different, a hydrogen atom, a lower alkyl group which may be substituted, an amino group which may be substituted or a carboxyl group which may be substituted, or $R_1$ and $R_2$ together with carbon atoms to which they are attached may form an aromatic ring which may contain a nitrogen atom, and a bond between the two carbon atoms to which $R_1$ and $R_2$ are attached is a single bond or a double bond, and Y is an oxygen atom, a sulfur atom or a group of $>NR_4$ wherein $R_4$ is a hydrogen atom or an alkyl group which may be substituted,

(3) a compound according to (1) above, in which B is a 6-membered heterocyclic group of the formula (VII):

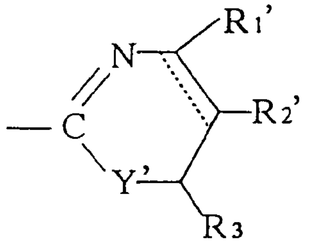

(VII)

wherein $R_1$' and $R_2$' are, the same or different, a hydrogen atom or a lower alkyl group, or $R_1$' and $R_2$' together with carbon atoms to which they are attached may form an aromatic ring which may contain a nitrogen atom, and a bond between the two carbon atoms to which $R_1$' and $R_2$' are attached is a single bond or a double bond, $R_3$ is a hydrogen atom, a hydrocarbon group or a heterocyclic group, and Y' is an oxygen atom, a sulfur atom or a group of $>NH$,

(4) a compound according to (1) above, in which the condensed imidazolyl group contains two to four nitrogen atoms as ring-constituting atoms,

(5) a compound according to (1) above, in which A' is an imidazolyl group condensed with a 6-membered heterocycle,

(6) a compound according to (1) above, in which A' is an imidazolyl group condensed with a 6-membered heterocycle containing nitrogen atom(s),

(7) a compound according to (1) above, in which A' is an imidazolyl group condensed with pyridine, pyridazine, pyrimidine, pyrazine or triazine,

(8) a compound according to (1) above, in which A' is an imidazolyl group condensed with pyridine or pyridazine,

(9) a compound according to (1) above, in which A' is a 2- or 3-nitro-imidazo[1,2-b]pyridazin-6-yl group,

(10) a compound according to (1) above, in which A' is a 2- or 3-nitro-imidazo[1,2-a]pyridin-5-yl group,

(11) a compound according to (2) above, in which $R_1$ and $R_2$ are, the same or different, a hydrogen atom or a substituted lower alkyl group,

(12) a compound according to (2) above, in which either of $R_1$ and $R_2$ is a lower alkyl group substituted by a mono- or di-lower alkyl-amino group,

(13) a compound according to (2) above, in which $R_1$ and $R_2$ are, the same or different, a straight-chain or branched alkyl group of 1 to 6 carbon atoms,

(14) a compound according to (1) above, in which X is a sulfur atom,

(15) a compound according to (2) above, in which Y is a group of $>NR_4$ wherein $R_4$ is a hydrogen atom or a substituted lower alkyl group,

(16) a compound according to (1) above, in which both of m and n are 0,

(17) a compound according to (1) above, which is 6-[(imidazol-2-yl)thio]-3-nitroimidazo[1,2-b]pyridazine, or its salt,

(18) a compound according to (1) above, which is 6-[[1-(2-hydroxyethyl)imidazol-2-yl]thio]-3-nitroimidazo[1, 2-b]pyridazine, or it salt,

(19) a compound according to (1) above, which is 6-[(4-dimethylaminomethylimidazol-2-yl)thio]-3-nitroimidazo[1,2-b]pyridazine, or its salt,

(20) an antibacterial agent which comprises an imidazole compound of the formula (I):

$$A\text{-}(CH_2)_m\text{-}X\text{-}(CH_2)_n\text{-}B \qquad (I)$$

wherein A is an imidazol-1-yl or condensed imidazolyl group having at least one nitro group on the imidazole ring, X is an oxygen atom or a sulfur atom, B is a 5- or 6-membered heterocyclic group which may be a condensed ring, and m and n are each an integer of 0 to 4, or its pharmaceutically acceptable salt and a carrier or diluent,

(21) an antibacterial agent according to (20) above, in which A in the formula (I) is a condensed imidazolyl group having at least one nitro group on the imidazole ring,

(22) an antibacterial agent according to (20) above, which exhibits antibacterial effect against the microorganism belonging to the genus *Helicobacter*,

(23) an antibacterial agent according to (20) above, which exhibits antibacterial effect against *Helicobacter pylori*, and

(24) an antiulcer agent which comprises an imidazole compound of the formula (I) as defined in (20) above or its pharmaceutically acceptable salt, and a carrier or diluent.

When the imidazole compounds of the above-mentioned formulae (I') and (I) have asymmetric carbon atoms, their corresponding optically-active compounds and racemate mixtures thereof are included in the present invention as well.

## PREFERRED EMBODIMENTS OF THE INVENTION

Details of each of the definitions for the formulae (I') and (I) are as follows.

When the term "lower" is used for the definitions for the formulae (I') and (I), it means a group having from one to six carbon atoms unless otherwise stated.

First, the definition for A' and A will be given in detail.

Examples of the "condensed imidazolyl group" represented by A' or A are an imidazolyl group which is condensed with 5- or 6-membered ring, preferably 6-membered ring. Examples of the 5- or 6-membered ring include carbocycles (e.g., aromatic ring or alicycle) and heterocycles (e.g., heterocycles which contain N, O or S), preferably heterocycles, more preferably heterocycles containing N.

Preferred examples of 5-membered carbocycles include cyclopentane and cyclopentene. Preferred examples of 6-membered carbocycles include cyclohexane, cyclohexene and benzene ring.

Preferred examples of the 5-membered heterocycles are a heterocycle containing one or two nitrogen atoms, such as pyrrole, imidazole, pyrazole, imidazolidine, imidazoline and so forth.

Preferred examples of the 6-membered heterocycles are a heterocycle containing one to three nitrogen atoms, such as pyridine, pyridazine, pyrimidine, pyrazine, triazine, piperazine and so forth. Among others pyridine and pyridazine are preferred.

Preferable examples of the condensed imidazolyl group are a condensed imidazolyl group containing two to four nitrogen atoms as the ring-constituting atoms. Specific examples of the condensed imidazolyl group are imidazo[1,2-b]pyridazinyl, imidazo[1,2-a]pyridyl, imidazo[1,5-a]pyridyl, imidazo[4,5-b]pyrazinyl, imidazo[1,5-a]pyrimidinyl, imidazo[1,2-d][1,2,4]triazinyl, imidazo[1,5-d][1,2,4]triazinyl, imidazo[5,1-c][1,2,4]triazinyl, imidazo[5,1-f][1,2,4]triazinyl, imidazo[1,5-b]pyridazinyl, 1H-purinyl, imidazo[1,5-a]pyridyl, 1H-imidazo[4,5-b]pyridyl, etc. Among others, the preferred examples are an imidazo[1,2-b]pyridazin-6 or -7 or -8-yl group and an imidazo[1,2-a]pyridin-5 or -6 or -7 or -8-yl group. More preferably, the condensed imidazolyl group is an imidazo[1,2-b]pyridazin-6-yl group and an imidazo[1,2-a]pyridin-5-yl group, most preferably, an imidazo[1,2-b]pyridazin-6-yl group.

The numbers of the nitro groups on the imidazole ring of the "condensed imidazolyl group" represented by A' or A or the "imidazol-1-yl group" represented by A are preferably from one to three or, more preferably, one. It is preferred that the nitro group is attached to a carbon atom which constitutes the imidazole ring.

Preferred examples of the "condensed imidazolyl group having at least one nitro group on the imidazole ring" represented by A or A' are a 2- or 3-nitroimidazo[1,2-b]pyridazin-6-yl group and a 2- or 3-nitroimidazo[1,2-a]pyridin-5-yl group, more preferably a 2- or 3-nitroimidazo[1,2-b]pyridazin-6-yl group.

The "condensed imidazolyl group" represented by A' or A may have a nitro group at the place which is other than the imidazole ring, i.e. on the ring which is condensed with imidazolyl group.

Preferred examples of the substituents which are other than nitro group on the "condensed imidazolyl group" represented by A' or A or the "imidazol-1-yl group" represented by A are a halogen atom (for example, chlorine, bromine, fluorine, iodine, etc.), hydroxyl group, carboxyl group, amino group, sulfo group, sulfamoyl group, mono- or di-lower alkyl-amino group, lower alkylcarbonylamino group, lower alkoxycarbonyl group, carbamoyl group, mono- or di-lower alkyl-carbamoyl group, lower alkyl group, lower alkoxy group, acyl group, etc.

Hereinafter, the definitions for the above-mentioned substituents which are other than the nitro group will be described in detail.

Preferred examples of the "mono- or di-lower alkylamino group" are amino groups which are substituted with one or two straight-chain or branched lower alkyl groups, specifically, such as methylamino, ethylamino, propylamino, isopropylamino, butylamino, isobutylamino, pentylamino, hexylamino, dimethylamino, diethylamino, etc.

Preferred examples of the "lower alkylcarbonylamino group" are straight-chain or branched lower alkylcarbonylamino groups, specifically, such as acetylamino, propionylamino, isopropionylamino, butyrylamino, etc.

Preferred examples of the "lower alkoxycarbonyl group" are straight-chain or branched lower alkoxycarbonyl groups, specifically, such as methoxycarbonyl, ethoxycarbonyl, propoxycarbonyl, isopropoxycarbonyl, butoxycarbonyl, isobutoxycarbonyl, etc.

Preferred examples of the "mono- or di-lower alkylcarbamoyl group" are the carbamoyl groups which are substituted with one or two, straight-chain or branched, lower alkyl group(s), specifically, such as methylcarbamoyl, ethylcarbamoyl, propylcarbamoyl, isopropylcarbamoyl, butylcarbamoyl, diethylcarbamoyl, dibutylcarbamoyl, pentylcarbamoyl, hexylcarbamoyl, etc.

Preferred examples of the "lower alkyl group" are straight-chain or branched $C_{1-6}$ alkyl groups, specifically, such as methyl, ethyl, n-propyl, isopropyl, n-butyl, isobutyl, tert-butyl, n-pentyl, n-hexyl, etc.

Preferred examples of the "lower alkoxy group" are straight-chain or branched $C_{1-6}$ alkoxy groups, specifically, such as methoxy, ethoxy, propoxy, isopropoxy, butoxy, isobutoxy, pentyloxy, hexyloxy, etc.

Preferred examples of the "acyl group" are $C_{1-6}$ acyl group, specifically, such as formyl, acetyl, propionyl, butyryl, isobutyryl, valeryl, isovaleryl, pivaroyl, hexanoyl, etc. Among those, formyl is particularly preferred.

The "condensed imidazolyl group" or "imidazol-1-yl group" represented by A' or A may have one or more of the same or different above-mentioned substituents which are other than nitro group. Preferable substituents on the imidazol-1-yl group represented by A which are other than nitro are lower alkyl groups.

When the condensed imidazolyl group represented by A' has substituent(s) which is other than nitro group, said substituent(s) may be attached to any atom(s) constituting the ring, preferably, carbon atom(s) constituting the ring.

Then the definition of B will be described in detail.

Preferred examples of the "five-membered heterocyclic group which may be a condensed ring" in the definition for B are the five-membered heterocyclic groups represented by the formula (VI):

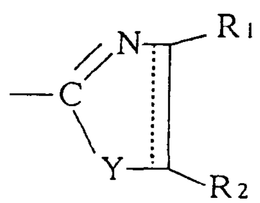

(VI)

in which $R_1$ and $R_2$ are, the same or different, a hydrogen atom, a lower alkyl group which may be substituted, an amino group which may be substituted or a carboxy group which may be substituted, or $R_1$ and $R_2$ together with the carbon atoms to which they are attached may form an aromatic ring which may contain a nitrogen atom, and a bond between the two carbon atoms to which $R_1$ and $R_2$ are attached is a single bond or a double bond (in the case of a single bond, two hydrogen atoms are attached thereto), and Y is an oxygen atom, a sulfur atom or a group of $>NR_4$ wherein $R_4$ is a hydrogen atom or an alkyl group which may be substituted.

Preferred examples of the "six-membered heterocyclic group which may be a condensed ring" are the six-membered heterocyclic groups represented by the formula (VII):

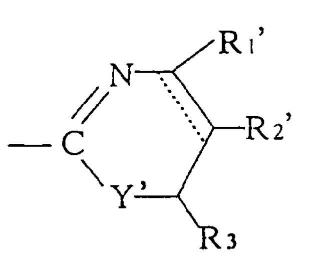

(VII)

in which $R_1$' and $R_2$' are, the same or different, a hydrogen atom or a lower alkyl group, or $R_1$' and $R_2$' together with carbon atoms to which they are attached may form an aromatic ring which may contain a nitrogen atom, and a bond between the two carbon atoms to which $R_1$' and $R_2$' are attached is a single bond or a double bond (in the case of a single bond, two hydrogen atoms are attached thereto), $R_3$ is a hydrogen atom, a hydrocarbon group or a heterocyclic group, and Y' is an oxygen atom, a sulfur atom or a group of >NH.

Each definitions in the above-mentioned five- and six-membered heterocyclic groups will be further illustrated below.

Preferred examples of the "lower alkyl group" in the definitions for $R_1$, $R_2$, $R_1$' or $R_2$' are straight-chain or branched $C_{1-6}$ alkyl groups, such as methyl, ethyl, propyl, isopropyl, butyl, isobutyl, pentyl, hexyl, etc, specifically $C_{1-3}$ alkyl groups such as methyl, ethyl, propyl, etc.

Examples of the substituent in the "lower alkyl group which may be substituted" in the definitions for $R_1$ and $R_2$ are the same as those which A or A' may have. Preferred examples of the above substituent are mono- or di-lower alkyl-amino groups.

$R_1$ or $R_1$' is preferably a hydrogen atom. $R_2$ or $R_2$' is preferably a hydrogen atom or a lower alkyl group which may be substituted by a mono- or di-lower alkyl-amino group.

Examples of the substituent in the "amino group which may be substituted" in the definitions for $R_1$ or $R_2$ are alkyl, cycloalkyl, aryl, aralkyl, heterocyclic groups and the like.

Preferred examples of the above alkyl groups are straight-chain or branched $C_{1-6}$ alkyl groups such as methyl, ethyl, n-propyl, isopropyl, n-butyl, isobutyl, tert-butyl, n-pentyl, n-hexyl, etc., more preferably, $C_{1-3}$ alkyl groups.

Preferred examples of the above cycloalkyl groups are $C_{3-3}$ cycloalkyl groups such as cyclopropyl, cyclobutyl, cyclopentyl, cyclohexyl, cycloheptyl, cyclooctyl, etc.

Preferred examples of the above aryl groups are $C_{6-12}$ aryl groups such as phenyl, naphthyl, biphenylyl, etc.

Preferred examples of the above aralkyl groups are $C_{7-19}$ aralkyl groups such as benzyl, phenethyl, benzhydryl, trityl, naphthylethyl, etc.

Preferred examples of the above heterocyclic groups are five- or six-membered heterocyclic groups containing 1 to 4 heteroatoms selected from nitrogen atom, oxygen atom and sulfur atom. Examples of the heterocyclic group include pyrrolidino, 2-oxopyrrolidino, pyrrolidinyl, pyrrolyl, pyrazolyl, imidazolyl, furyl, thienyl, oxazolyl, isoxazolyl, isothiazolyl, thiazolyl, piperidino, piperidinyl, pyridyl, pyridazinyl, pyrazinyl, piperazinyl, pyrimidinyl, indolyl, 1,2,3-triazolyl, 1,2,4-triazolyl, 1,3,4-triazolyl, tetrazolyl, 1,3-dioxoranyl, morpholino, morpholinyl and the like. The heterocyclic group may form a condensed bicyclic group (e.g., 8-quinolyl, 8-purinyl, etc.) with a five- or six-membered ring (e.g., benzene, pyridine, cyclohexane, etc.).

The above alkyl as well as the above cycloalkyl, aryl, aralkyl, heterocyclic groups may further be substituted with 1 to 3 appropriate substituents (e.g., hydroxyl, carboxyl, amino optionally substituted with $C_{1-6}$ alkyl, etc.).

Preferred examples of the substituent in the "amino group which may be substituted" in the definition for $R_1$ or $R_2$ are $C_{1-6}$ alkyl groups, more preferably $C_{1-3}$ alkyl groups.

Examples of the "carboxyl group which may be substituted" in the definitions for $R_1$ or $R_2$ are a carboxyl group and its derivatives such as esters, amides, etc.

Examples of the above esters are alkyl esters, cycloalkyl esters, arylesters, aralkylesters, heterocyclic alkyl esters, etc.

Examples of the above amides are unsubstituted amide and mono- or di-substituted amide, etc.

Examples of the substituent in the above subsitituted amide are alkyl, cycloalkyl, aryl, aralkyl, heterocyclic alkyl, etc., which may be substituted by carboxy or amino-lower alkyl. Examples of these alkyl, cycloalkyl, aryl, aralkyl, heterocyclic groups above-mentioned are the same as those in the definitions above-mentioned. When the amide is di-substituted amide, it may form an ring with nitrogen atom to which they are attached. Examples of the ring are six-membered rings containing one or two nitrogen atom(s), such as pyridine, pyrazine, etc. The nitrogen-containing ring may have substituents, such as lower alkyl or aralkyl.

Preferred examples of the "hydrocarbon group" in the definition for $R_3$ are hydrocarbon groups having 1-20 carbon atoms such as a lower alkyl group, aryl group, arylalkyl group, etc. Among others, the aryl group is particularly preferred.

Preferred examples of the above-mentioned "lower alkyl group" are the same as those in the definitions for $R_1$, $R_2$, $R_1$' or $R_2$'.

Examples of the above-mentioned "aryl group" are the aryl groups having 6 to 12 carbon atoms. Preferred examples are phenyl, tolyl, xylyl, cumenyl, mesityl, biphenyl, naphthyl, etc. Among others, the particularly preferred ones are phenyl, tolyl, etc.

Preferred examples of the above-mentioned "arylalkyl group" are benzyl, phenethyl, etc.

Examples of the "heterocyclic group" in the definition for $R_3$ are the same as those in the definitions of subsituents for $R_1$ or $R_2$. Preferred examples are pyridylmethyl, pyridylethyl, pyridyl, furfuryl, etc.

Examples of the "alkyl group" in the definition for $R_4$ are $C_{1-8}$ straight-chain or branched alkyl groups, such as methyl, ethyl, propyl, butyl, pentyl, hexyl, heptyl, octyl, isopropyl, isobutyl, neopentyl, t-butyl, etc. Preferred examples are $C_{1-3}$ straight-chain or branched alkyl groups, such as methyl, ethyl, propyl, etc.

Examples of the substituent in the "alkyl group which may be substituted" in the definition for $R_4$ are aromatic cyclic group, one to three hydroxyl group(s) and one to three amino group(s) which may be substituted. Examples of the aromatic rings of the aromatic cyclic group are $C_{6-12}$ aromatic rings which may containe one to three nitrogen atom(s), such as pyridine, pyridazine, pyrimidine, pyrazine, triazine, quinoline, isoquinoline, benzene, naphthalene, etc. These $C_{6-12}$ aromatic rings may be substituted by $C_{1-6}$ alkyl groups such as methyl, ethyl, propyl, butyl, isopropyl, etc., $C_{1-6}$ alkoxy groups such as methoxy, ethoxy, propoxy, butoxy, isopropoxy, etc., or aromatic cyclic groups such as phenyl, pyradinyl, etc. Examples of the amino groups which may be substituted are methylamino, ethylamino, propylamino, dimethylamino, diethylamino, methylbenzylamino, pyrolidino, piperidino, etc.

Preferred examples of the substituent in the "alkyl group which may be substituted" in the definition for $R_4$ are hydroxyl group(s).

$R_4$ is preferably a hydrogen atom or a $C_{1-3}$ alkyl group which may be substituted.

When $R_1$ and $R_2$ together with carbon atoms to which they are attached form an aromatic ring which may contain one or two nitrogen atom(s), their examples are six-membered aromatic rings such as benzene, pyridine, pyrazine, etc. The particularly preferred example is benzene.

Y is preferably a group of $>NR_4$. Y' is preferably a group of $>NH$.

The five- or six-membered heterocyclic group which may be a condensed ring represented by B may be optionally substituted. Examples of the substituents are the same as those which A or A' may have.

B is preferably a five-membered heterocyclic group which may be a condensed ring. More preferably, B is a five-membered hetrocyclic group respresented by the formula (VI) wherein $R_1$ is a hydrogen atom, a carboxyl group substituted by a lower alkyl group or a group of

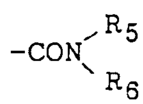

in which $R_5$ is a hydrogen atom, $R_6$ is a lower alkyl group which is substituted by amino or carboxyl group(s), or $R_5$ and $R_5$ together with nitrogen atom to which they are attached may form a six-membered heterocycle which may further contain one nitrogen atom, the heterocycle optionally being substituted by a lower alkyl group which may be substituted by phenyl,

$R_2$ is a hydrogen atom or a lower alkyl group which may be substituted by a di-lower alkyl-amino group, or $R_1$ and $R_2$ together with carbon atoms to which they are attached may form a six-membered aromatic ring which may contain a nitrogen atom, and

Y is an oxygen atom, a sulfur atom or a group of $>NR_7$ in which $R_7$ is a hydrogen atom or a $C_{1-8}$ alkyl group which may be substituted by a hydroxy group, a phenyl group which may be substituted by lower alkyl, lower alkoxy-phenyl or a nitrogen-containing six-membered heterocyclic group which may be condensed with a benzene ring.

Most preferably, B is a five-membered heterocyclic group represented by the formula (VI) wherein $R_1$ is a hydrogen atom, $R_2$ is a hydrogen atom or a lower alkyl group substituted by a di-lower alkyl-amino group, and

Y is a group of $>NR_7'$ in which $R_7'$ is a hydrogen atom or a $C_{1-3}$ alkyl group substituted by a hydroxy group.

In the definition for the formulae (I') and (I), a sulfur atom is preferred for X; an integer of 0 to 2 is preferred for m, 0 is more preferred for m; and 0 is particularly preferred for n.

Preferred examples of the compound of the formula (I') are 6-[(imidazol-2-yl)thio]-3-nitroimidazo[1,2-b]pyridazine (compound prepared in Ex. 6-3), 6-[[1-(2-hydroxyethyl) imidazol-2-yl]thio]-3-nitroimidazo[1,2-b]pyridazine (compound prepared in Ex. 9-8), and 6-[(4-dimethylaminomethylimidazol-2-yl)thio]-3-nitroimidazo[1,2-b]pyridazine (compound prepared in Ex. 11), or salts thereof.

When the imidazole compound represented by the formulae (I') or (I) [hereinafter, they are sometimes referred to as the compound (I') and (I), respectively] has a group exhibiting a basicity or an acidity in its molecule, it may form a salt with an acid or with an alkali. The kinds of the salts are not particularly limited. Examples of the salt when a group exhibiting a basicity is present are inorganic acid salts such as hydrochloride, hydrobromide, sulfate, nitrate, phosphate, etc. and organic acid salts such as acetate, tartrate, citrate, fumarate, maleate, toluenesulfonate, methanesulfonate, etc. Examples of the salt when a group exhibiting an acidity is present are salts with an alkali metal such as sodium and potassium. Usually the above-mentioned salts may be used as the pharmaceutically acceptable salts.

The compounds (I') of the present invention may, for example, be prepared according to the following method. Thus,

(1) a compound represented by the formula (II'):

$$A'-(CH_2)_m-Z \qquad (II')$$

in which the symbols have the same meanings as defined above and Z is a reactive group, or its salt is allowed to react with a compound represented by the formula (III):

$$H-X-(CH_2)_n-B \qquad (III)$$

in which the symbols have the same meanings as defined above, or its salt; or

(2) a compound represented by the formula (IV'):

$$A'-(CH_2)_m-X-H \qquad (IV')$$

in which the symbols have the same meanings as defined above, or its salt is allowed to react with a compound represented by the formula (V):

$$Z-(CH_2)_n-B \qquad (V)$$

in which the symbols have the same meanings as defined above and Z is a reactive group, or its salt under the reaction conditions as given below.

With regard to the reactive group represented by the symbol Z in the definitions for the formulae (II') and (V), any reactive group which is commonly used in the art may be used. Preferred examples of the reactive group are a halogen atom (e.g., chlorine, bromine, fluorine and iodine), $C_6$ to $C_{10}$ arylsulfonyloxy group (e.g., benzenesulfonyloxy, p-tolylsulfonyloxy, etc.), $C_1$ to $C_4$ alkylsulfonyloxy (e.g., methanesulfonyloxy, etc.), etc.

Preferred examples of each of the definitions (except Z) for the formulae (II'), (III), (IV') and (V) are the same as those for the formula (I').

The reaction may be carried out in a solvent such as ethers (e.g., diethyl ether, tetrahydrofuran, dioxane, etc.), halogenide hydrocarbons (e.g., dichloromethane, dichloroethane, chloroform, carbon tetrachloride, etc.), hydrocarbons (e.g., benzene, toluene, hexane, heptane, etc.), dimethyl formamide, dimethyl sulfoxide, etc.

If necessary, this reaction may be carried out in the presence of a base as well. Examples of such a base are an organic base (e.g., 4-dimethylaminopyridine, triethylamine, triethylenediamine and tetramethylethylenediamine), an inorganic base (e.g., sodium bicarbonate, potassium bicarbonate, sodium carbonate, potassium carbonate, sodium hydroxide and potassium hydroxide), sodium hydride, potassium hydride, etc.

The suitable reaction temperature for this reaction is about -50°C to about 100°C and, preferably, about 0°C to about 50°C. The reaction time is usually about 1 to about 48 hours and, preferably, about 5 to about 10 hours.

In carrying out the present reaction, it is suitable to use about 1 to 10 moles, preferably about 1 to 3 moles, of the compound represented by the formula (III) or (IV') to one mole of the compound represented by the formula (II') or (V).

The reaction product which is synthesized by the present reaction may be isolated/purified by known means such as extraction with solvent, change of alkalinity or acidity, dissolution into a different solvent, salting-out, crystallization, recrystallization, chromatography, etc.

The compound (I') obtained by the above-mentioned reaction may be converted, if desired, to a salt thereof by conventional means which are usually applied in the art.

The salts for the starting materials (II'), (III), (IV') and (V) used for the synthesis of the compound (I') may be the same salts as those applied to the compound (I').

The starting materials (II'), (IV), (IV') and (V) used for the synthesis of the above-given compound (I') may be synthesized by a method known per se, a method given in the following examples or a method similar thereto.

The compound (I) of the present invention may also be manufactured by the reaction of:

(1) a compound represented by the formula (II):

$$A-(CH_2)_m-Z \qquad (II)$$

in which the symbols have the same meanings as defined above, or its salt with the above-mentioned compound (III) or its salt; or

(2) a compound represented by the formula (IV):

$$A-(CH_2)_m-X-H \qquad (IV)$$

in which the symbols have the same meanings as defined above, or its salt with the above-mentioned compound (V) or its salt,

under the same reaction condition for the synthesis of the above-mentioned compound (I').

The compound (I) of the present invention or its salt has an antibacterial activity and, particularly, a high antibacterial activity against the microorganisms belonging to the genus *Helicobacter* represented by *Helicobacter pylori*. Accordingly, it is useful as an antibacterial agent in preventing or treating various gastrointestinal diseases (e.g., duodenal ulcer, gastric ulcer, chronic gastritis, etc.) caused by *Helicobacter pylori* as mentioned hereinabove.

Further, the compound (I) of the present invention or its salt has an antibacterial activity, in addition to the microorganisms belonging to the genus *Helicobacter*, against Gram-positive bacteria such as *Staphylococcus aureus* and Gram-negative bacteria such as *Hemophilus influenzae* and *Moraxella catarrhalis*.

Furthermore, the compound (I) of the present invention or its salt is also effective for prevention or treatment of ulcers which are not caused by *Helicobacter pylori* such as stress ulcer, and thus is effective as a novel antiulcer agent for preventing or treating duodenal ulcer, gastric ulcer, etc.

The compound (I) of the present invention or its pharmaceutically acceptable salt can be administered as an antibacterial agent or an antiulcer agent to mammals including human being either by an oral route or a parenteral route. Usually, an oral administration is preferred.

Examples of the pharmaceutical preparations for oral administration are tablets (including sugar-coated tablets and film-coated tablets), pills, granules, diluted powders, capsules (including soft capsules), sirups, emulsions, suspensions, etc. Examples of the preparations for parenteral administration are injections, transfusions, infusions, suppositories, etc.

With regard to a method for the synthesis of the above-mentioned preparations from the compound (I) of the present invention or its salt, per se known methods which are commonly used in the art may be applied. If necessary in the synthesis of such preparations, fillers, binders, disintegrators, lubricants, sweeteners, surface-active agents, suspending agents, emulsifiers, etc. which are commonly used in the art for the synthesis of such preparations may be compounded.

In the synthesis of the tablets, for example, of the compound (I) of the present invention or its pharmaceutically acceptable salt, fillers, binders, disintegrators, lubricants, etc. may be compounded. In the synthesis of the pills and the granules, fillers, binders, disintegrators, etc. may be compounded. In preparing the diluted powders and the capsules, fillers may be compounded; in preparing the sirups, sweeteners may be compounded; and in preparing the emulsions and the suspensions, suspending agents, surface-active agents, emulsifiers, etc. may be compounded. Examples of the fillers are lactose, white sugar, glucose, starch, sucrose, microcrystalline cellulose, licorice root powder, mannitol, sodium bicarbonate, calcium phosphate, calcium sulfate, etc. Examples of the binders are 5-10 wt% starch paste solution, 10-20 wt% gum arabic solution or gelatin solution, 1-5 wt% tragacanth solution, caroxymethylcellulose solution, sodium alginate solution, glycerol, etc. Examples of the disintegrators are starch, calcium carbonate, etc. Examples of the lubricants are magnesium stearate, stearic acid, calcium stearate, pure talc, etc. Examples of the sweeteners are glucose, fructose, invert sugar, sorbitol, xylitol, glycerol, simple sirup, etc. Examples of the surface-active agents are sodium laurylsulfate, polysorbate 80, sorbitan monofatty acid esters, polyoxyl 40 stearate, etc. Examples of the suspending agents are gum arabic, sodium alginate, sodium carboxymethylcellulose, methylcellulose, bentonite, etc. Examples of the emulsifiers are gum arabic, tragacanth, gelatin, polysorbate 80, etc.

If desired, moreover, in the synthesis of the above-mentioned preparations of the compound (I) of the present invention or its pharmaceutically acceptable salt, coloring agents, preservatives, aromatic agents, flavoring agents, stabilizers, thickeners, etc. which are commonly used in the art of pharmaceutical preparations may be compounded.

The compound (I) of the present invention or a pharmaceutically acceptable salt is stable and with low toxicity and can be used safely. Its dose per day may vary depending upon the state and the body weight of the patient, the kind of the particular compound, the route of administration, etc. In the case of an oral administration, the dose for an adult is suitably about 1 to about 500 mg or, preferably, about 10 to about 200 mg per day. Within the above-mentioned dose range, no toxicity is noted.

Further, the compound (I) of the present invention or its pharmaceutically acceptable salt may be administered together with other antibacterial agents and antiulcer agents.

The present invention will be more specifically illustrated by way of the following Examples, Preparation Examples and Test Examples. But it should be noted that the present invention is not limited thereto.

Example 1

Synthesis of 1-(benzoxazol-2-yl)thioethyl-2-methyl-5-nitroimidazole.

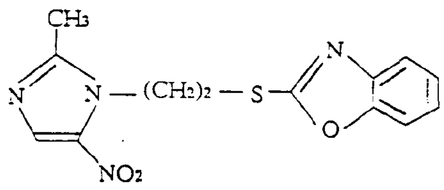

(1) 2-Methyl-5-nitro-1-[2-(toluenesulfonyloxy)ethyl]imidazole.

2-Methyl-5-nitro-1-imidazoleethanol (5.2 g), 5.7 g of p-toluenesulfonyl chloride and 3.4 g of triethylamine were dissolved in 100 ml of dichloromethane and the solution was stirred at 40°C overnight. After washing with water, it was dried over anhydrous sodium sulfate, and the solvent was evaporated therefrom. Ethyl ether was added to the filtrate followed by filtration to give 8.9 g of crystals of 2-methyl-5-nitro-1-[2-(toluenesulfonyloxy) ethyl]imidazole.

(2) 1-(Benzoxazol-2-yl)thioethyl-2-methyl-5-nitroimidazole.

First, sodium hydride (60 wt% content) (0.132g) was added to 10 ml of dimethyl formamide, and the mixture was stirred at room temperature for ten minutes. 2-Mercaptobenzoxazole (0.45g) was added to the mixture, which was stirred at room temperature for 30 minutes. 5-Methyl-2-nitro-1-[2-(toluenesulfonyloxy)ethyl]imidazole (0.97g) was further added thereto and stirred at 60°C overnight.

After adding 100 ml of water to the reaction solution, the mixture was extracted with dichloromethane. The extract was dried over anhydrous sodium sulfate, and evaporated to remove the solvent. The residue was recrystallized from dichloromethane/ethyl ether to give 0.80 g of 1-(benzoxazol-2-yl)thioethyl-2-methyl-5-nitroimidazole.

M.p.: 144-145°C

| Elemental analysis for $C_{13}H_{12}N_4O_3S$ | | | |
|---|---|---|---|
| Calculated: | C 51.31; | H 3.97; | N 18.41 |
| Found: | C 51.15; | H 4.04; | N 18.34 |

Example 2

Synthesis of 1-(benzimidazol-2-yl)thioethyl-2-methyl-5-nitroimidazole.

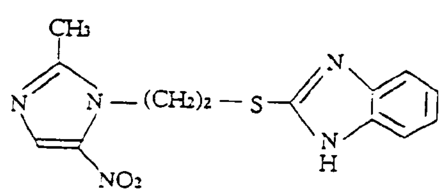

2-Mercaptobenzimidazole (0.45 g), which was used instead of 0.45 g of 2-mercaptobenzoxazole, was reacted with 2-methyl-5-nitro-1-[2-(toluenesulfonyloxy)ethyl]imidazole by the same manner as in Example 1 to give 0.52 g of the title compound.

M.p.: 290-292°C (decomp.)

| Elemental analysis for $C_{13}H_{13}N_5O_2S \cdot 1/2H_2O$ | | | |
|---|---|---|---|
| Calculated: | C 49.99; | H 4.52; | N 22.42 |
| Found : | C 50.31; | H 4.48; | N 22.71 |

Example 3

Synthesis of 1-(benzothiazol-2-yl)thioethyl-2-methyl -5-nitroimidazole.

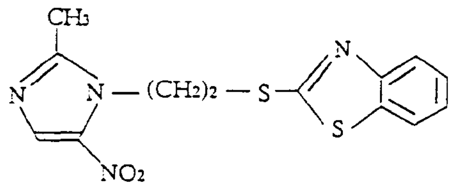

2-Mercaptobenzothiazole (0.45 g), which was used instead of 0.45 g of 2-mercaptobenzoxazole, was reacted with 2-methyl-5-nitro-1-[2-(toluenesulfonyloxy)ethyl]imidazole by the same manner as in Example 1 to give 0.61 g of the title compound.

M.p.: 121-122°C

| Elemental analysis for $C_{13}H_{12}N_4O_2S_2$ | | | |
|---|---|---|---|
| Calculated: | C 48.73; | H 3.78; | N 17.49 |
| Found: | C 48.71; | H 3.79; | N 17.42 |

Example 4

Synthesis of 6-(benzoxazol-2-yl)thio-3-nitroimidazo [1,2-b]pyridazine.

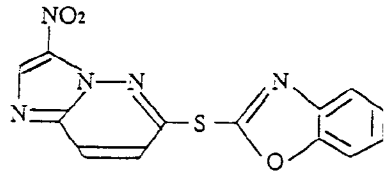

Sodium hydride (60% content) (0.132 g) was suspended in 10 ml of dimethylformamide, to which 0.45 g of 2-mercaptobenzoxazole was added. The mixture was stirred at room temperature for ten minutes. 6-Chloro-3-nitroimidazo[1,2-b] pyridazine (0.6g) was added thereto and the mixture was stirred at 120°C for ten hours. After adding 100 ml of water, the mixture was extracted with dichloromethane. The extract was dried over anhydrous sodium sulfate, and evaporated to remove the solvent. The residue was recrystallized from dichloromethane to give 0.56 g of 6-(benzoxazol-2-yl)thio-3-nitroimidazo [1,2-b]pyridazine as crystals.

M.p.: 176-178°C

| Elemental analysis for $C_{13}H_7N_5O_3S$ | | | |
|---|---|---|---|
| Calculated: | C 49.84; | H 2.25; | N 22.35 |
| Found: | C 49.72; | H 2.05; | N 21.97 |

Example 5

Synthesis of 6-(benzimidazol-2-yl)thio-3-nitroimidazo[1,2-b]pyridazine.

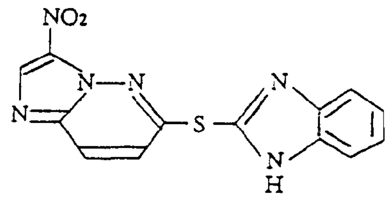

2-Mercaptobenzimidazole (0.45 g), which was used instead of 0.45 g of 2-mercaptobenzoxazole, was reacted with 2-mercaptobenzimidazole by the same manner as in Example 4 to give 0.60 g of the title compound.

M.p.: 283-285°C

| Elemental analysis for $C_{13}H_8N_6O_2S$ | | | |
|---|---|---|---|
| Calculated: | C 50.00; | H 2.58; | N 26.91 |
| Found: | C 50.13; | H 2.41; | N 26.73 |

Example 6

The same operation as in Example 4 was carried out using 6-chloro-3-nitroimidazo[1,2-b]pyridazine to give the compounds which are listed in Table 1.

[Table 1]

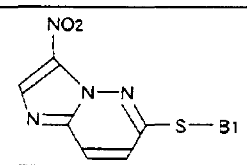

| Ex. Nos. | $B_1$ | Molecular Formula (Molecular Weight) | Melting Point (°C) | Elemental Analysis Calculated (Found) C | H | N |
|---|---|---|---|---|---|---|
| 6-1 | 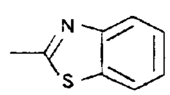 | $C_{13}H_7N_5O_2S_2$ (329.36) | 210-211 | 47.41 (47.22 | 2.14 2.36 | 21.26 21.20) |
| 6-2 | 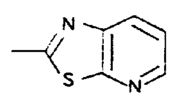 | $C_{12}H_6N_6O_2S_2$ (330.34) | Noncrystalline solid | 43.63 (43.51 | 1.83 2.06 | 25.44 25.33) |

[Table 1] (continued)

| Ex. Nos. | $B_1$ | Molecular Formula (Molecular Weight) | Melting Point (°C) | Elemental Analysis Calculated (Found) C | H | N |
|---|---|---|---|---|---|---|
| 6-3 | 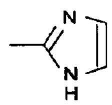 | $C_9H_6N_6O_2S$ (262.24) | 229-230 | 41.22 (41.23 | 2.31 2.29 | 32.05 32.35) |
| 6-4 | 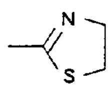 | $C_9H_7N_5O_2S_2$ (281.31) | 223-225 | 38.43 (38.43 | 2.51 2.54 | 24.89 25.09) |
| 6-5 | 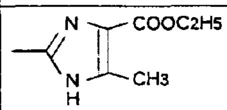 | $C_{13}H_{12}N_6O_4S \cdot HCl$ (384.80) | 188-189 | 40.58 (40.89 | 3.41 3.10 | 21.84 22.02) |

Example 7

The same operation as in Example 4 was carried out using 5-chloro-3-nitroimidazo[1,2-a]pyridine to give the compounds listed in Table 2.

[Table 2]

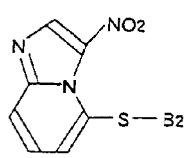

| Ex. Nos. | $B_2$ | Molecular Formula (Molecular Weight) | Melting Point (°C) | Elementary Analysis Calculated (Found) C | H | N |
|---|---|---|---|---|---|---|
| 7-1 | 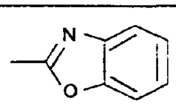 | $C_{14}H_8N_4O_3S$ (312.30) | 148-149 | 53.84 (53.90 | 2.58 2.52 | 17.94 17.93) |
| 7-2 | 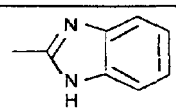 | $C_{14}H_9N_5O_2S$ (311.32) | 243-244 | 54.01 (54.25 | 2.91 2.94 | 22.50 23.03) |

[Table 2] (continued)

| Ex. Nos. | $B_2$ | Molecular Formula (Molecular Weight) | Melting Point (°C) | Elementary Analysis Calculated (Found) C H N |
|---|---|---|---|---|
| 7-3 | 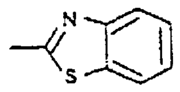 | $C_{14}H_8N_4O_2S_2$ (328.36) | 184-185 | 51.21 2.46 17.06 (51.05 2.40 17.23) |
| 7-4 | 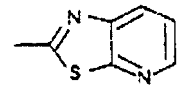 | $C_{13}H_7N_5O_2S_2$ (329.35) | 138-139 | 47.41 2.14 21.26 (47.29 2.09 21.32) |

Example 8

Synthesis of 1-benzyl-2-[(3-nitroimidazo[1,2-b]pyridazin-6-yl)thio]imidazole monohydrochloride.

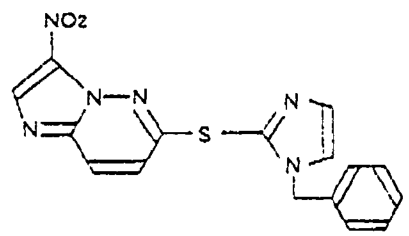

2-[(3-Nitroimidazo[1,2-b)pyridazin-6-yl)thio]imidazole (262 mg) prepared in Example 6-3 was added to a suspension of 40 mg of 60% sodium hydride in 20 ml of dimethylformamide at 0°C and the mixture was stirred for ten minutes to which was added benzylbromide (170mg) and the mixture was stirred at room temperature for 3 hrs. To the reaction solution was added 100 ml of water. The mixture was extracted with dichloromethane. The extract was dried over anhydrous magnesium sulfate and evaporated to remove the solvent. The residue was purified by means of a silica gel column chromatography. The resulting solid was dissolved in dichloromethane, to which then 1 ml of solution of 4N hydrogen chloride in ethyl acetate was added. The solvent was evaporated therefrom and the residue was recrystallized from a mixture of ethanol and ethyl ether to give 154 mg of 1-benzyl-2-[(3-nitroimidazo[1,2-b]pyridazin-6-yl)thio]imidazole monohydrochloride, colorless crystals, m.p. 147-150°C.

| Elemental analysis for $C_{16}H_{12}N_6O_2S \cdot HCl$ | | | |
|---|---|---|---|
| Calculated: | C 49.42; | H 3.37; | N 21.61 |
| Found: | C 49.11; | H 3.27; | N 21.64 |

Example 9

The same operation as in Example 8 was carried out using 2-[(3-nitroimidazo[1,2-b]pyridazin-6-yl)thio]imidazole obtained in Example 6 to give the compounds listed in Table 3.

[Table 3]

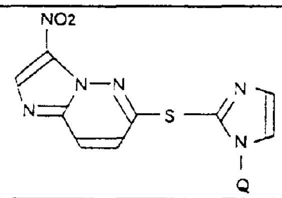

| Ex. Nos. | Q | Molecular Formula (Molecular Weight) | Melting Point (°C) | Elemental Analysis Calculated (Found) C | H | N |
|---|---|---|---|---|---|---|
| 9-1 | 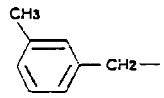 | $C_{17}H_{14}N_6O_2S$ ·HCl (402.86) | 162-163 | 50.68 (50.34 | 3.75 3.69 | 20.86 20.83) |
| 9-2 | 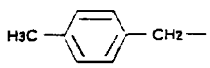 | $C_{17}H_{14}N_6O_2S$ ·HCl·1/2$H_2O$ (411.87) | 164-166 | 49.58 (49.61 | 3.92 3.84 | 20.40 20.09) |
| 9-3 | 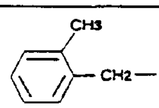 | $C_{17}H_{14}N_6O_2S$ ·HCl (402.86) | 130-132 | 50.68 (50.84 | 3.75 3.79 | 20.86 20.43) |

[Table 3] (continued)

| Ex. Nos. | Q | Molecular Formula (Molecular Weight) | Melting Point (°C) | Elemental Analysis Calculated (Found) C | H | N |
|---|---|---|---|---|---|---|
| 9-4 | 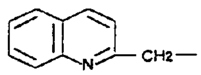 | $C_{19}H_{13}N_7O_2S$ ·2HCl (476.35) | Noncrystalline Solid | 46.16 (45.92 | 3.47 3.36 | 19.83 19.93) |
| 9-5 | 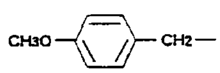 | $C_{17}H_{14}N_6O_3S$ ·HCl (418.86) | 159-161 | 48.75 (48.28 | 3.61 3.57 | 20.06 19.85) |
| 9-6 | 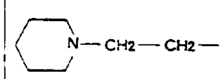 | $C_{15}H_{17}N_7O_2S$ ·2HCl (432.33) | Noncrystalline Solid | 41.67 (41.71 | 4.43 4.42 | 22.68 22.54) |
| 9-7 | 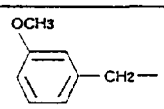 | $C_{17}H_{14}N_6O_3S$ ·HCl (418.86) | 179-181 | 48.75 (48.37 | 3.61 3.64 | 20.06 20.12) |
| 9-8 | $HO(CH_2)_2$— | $C_{11}H_{10}N_6O_3S$ (306.31) | 172-174 | 43.13 (43.00 | 3.29 3.29 | 27.44 27.11) |
| 9-9 | 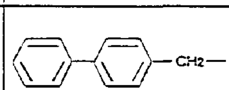 | $C_{22}H_{16}N_6O_2S$ (464.94) | 171-173 | 56.83 (56.63 | 3.69 3.71 | 18.08 17.96) |
| 9-10 | $CH_3$— | $C_{10}H_8N_6O_2S$ ·2HCl (349.20) | 224-226 | 34.40 (33.98 | 2.89 2.87 | 24.07 23.92) |
| 9-11 | 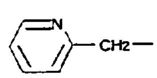 | $C_{15}H_{11}N_7O_2S$ ·2HCl (426.29) | Noncrystalline Solid | 42.26 (42.17 | 3.07 3.08 | 23.00 22.91) |
| 9-12 | $CH_3(CH_2)_7$— | $C_{17}H_{22}N_6O_2S$ ·HCl·$H_2O$ (428.95) | 133-135 | 47.60 (47.65 | 5.87 5.78 | 19.58 20.08) |
| 9-13 | 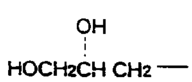 | $C_{12}H_{12}N_6O_4S$ ·HCl (372.79) | 193-196 | 38.66 (38.51 | 3.52 3.51 | 22.54 22.67) |

Example 10

Synthesis of 6-(benzimidazol-2-yl)oxy-3-nitroimidazo-[1,2-b]pyridazine.

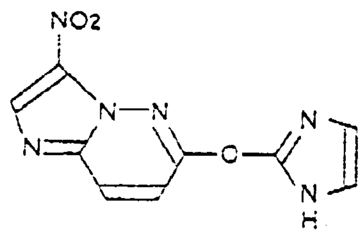

Sodium hydride (60%) (0.12 g) was added to a solution of 0.8 g of 2-hydroxybenzimidazole and 0.6 g of 6-chloro-3-nitroimidazo[1,2-b]pyridazine in 20ml of dimethylformamide. The mixture was stirred at room temperature for two days. After evaporation of the solvent therefrom, the residue was washed with water, methanol and dimethyl sulfoxide to give 0.22 g of 6-(benzimidazol-2-yl)oxy-3-nitroimidazo[1,2-b]pyridazine, colorless crystals, m.p. 322-323°C.

| Elemental analysis for $C_{13}H_8N_6O_3$ | | | |
|---|---|---|---|
| Calculated: | C 52.71; | H 2.72; | N 28.37 |
| Found: | C 53.12; | H 2.82; | N 28.14 |

Example 11

Synthesis of 6-[(4-dimethylaminomethylimidazol-2-yl)thio]-3-nitroimidazo[1,2-b]pyridazine trihydrochloride.

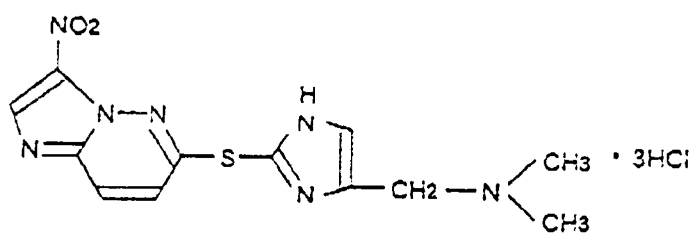

Dimethylamine aqueous solution (50%, 990 mg) was cooled at 0°C and 1 ml of acetic acid and 1.2 g of 37% formaldehyde were added thereto. To the resulting solution was added 0.26 g of 2-[(3-nitroimidazo[1,2-b]pyridazin-6-yl)thio] imidazole (the compound prepared in Example 6-3) and the mixture was stirred for 48 hours at 50°C. To the reaction solution was added 20 ml of 1N sodium hydroxide aqueous solution and the mixture was extracted with dichloromethane. The extract was dried over anhydrous magnesium sulfate and evaporated to remove the solvent. The residue was purified by means of a silica gel column chromatography followed by adding 1 ml of 4N hydrogen chloride solution in ethyl acetate thereto. After evaporating the solvent, the precipitated crystals were suspended in dichloromethane and filtered to give 52mg of 6-[(4-dimethylaminomethylimidazol-2-yl)thio]-3-nitroimidazo[1,2-b]pyridazin trihydrochloride, colorless crystals, m.p. 175-177°C.

| Elemental analysis for $C_{12}H_{13}N_7O_2S{\cdot}3HCl$ | | | |
|---|---|---|---|
| Calculated: | C 33.62; | H 3.76; | N 22.87 |
| Found: | C 33.54; | H 3.89; | N 22.37 |

Example 12

Synthesis of 5-methyl-4-(4-methylpiperazinocarbonyl)-2-[(3-nitroimidazo[1,2-b]pyridazin-6-yl)thio]imidazole dihydrochloride.

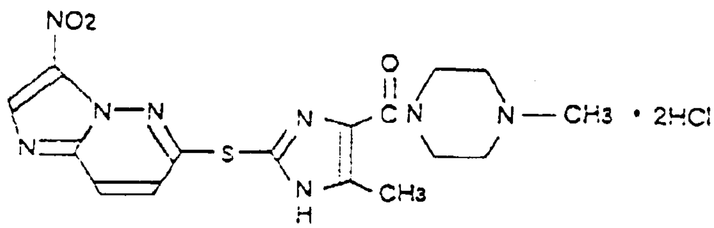

Diethyl cyanophosphate (196 mg) was added to a solution of 158 mg of 4-carboxy-5-methyl-2-mercaptoimidazole, 100 mg of N-methylpiperazine and 100 mg of triethylamine in 10 ml of dimethylformamide and the mixture was stirred at 0°C for 30 minutes. After adding 5 ml of water, the mixture was further stirred at room temperature for 30 minutes and evaporated to remove the solvent. The resulted solid was suspended in ethyl ether and filtered. The collected one (248 mg) was subjected to the same operation as in Example 4 to obtain 5-methyl-4-(4-methylpiperazinocarbonyl)-2-[(3-nitroimidazo[1,2-b]pyridazin-6-yl)thio]imidazole (30 mg). This was converted to the corresponding hydrochloride as colorless crystals of m.p. 194-196°C (43mg).

| Elemental analysis for $C_{16}H_{18}N_8O_3S{\cdot}2HCl$ | | | |
|---|---|---|---|
| Calculated: | C 43.79; | H 4.36; | N 25.53 |
| Found: | C 43.92; | H 4.43; | N 25.32 |

Example 13

Synthesis of 4-(4-benzylpiperazinocarbonyl)-5-methyl-2-[(3-nitroimidazo[1,2-b]pyridazin-6-yl)thio]imidazole dihydrochloride.

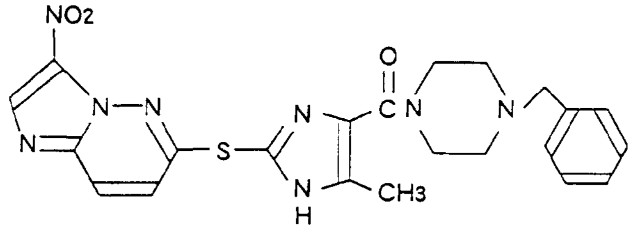

The same operation as in Example 12 was carried out using 4-carboxy-5-methyl-2-mercaptoimidazole to give 4-(4-benzylpiperazinocarbonyl)-5-methyl-2-[(3-nitroimidazo[1,2-b]pyridazin-6-yl)thio]imidazole dihydrochloride, colorless crystals, m.p. 178-180°C.

| Elemental analysis for $C_{22}H_{22}N_8O_3S{\cdot}2HCl{\cdot}1/2H_2O$ | | | |
|---|---|---|---|
| Calculated: | C 47.15; | H 4.50; | N 19.99 |
| Found: | C 46.89; | H 4.71; | N 19.51 |

Example 14

Synthesis of 4-[(5-amino-1-carboxy)pentylaminocarbonyl]-5-methyl-2-[(3-nitroimidazo[1,2-b]pyridazin-6-yl) thio]imidazole dihydrochloride.

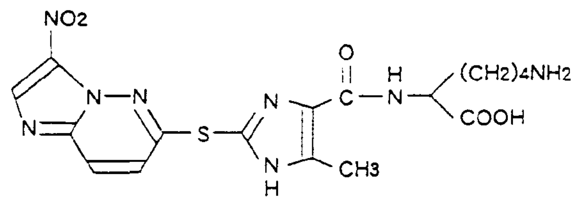

The same operation as in Example 12 was carried out using 316 mg of 4-carboxy-5-methyl-2-mercaptoimidazole and 604 mg of $N^{\varepsilon}$-tert-butoxycarbonyllysine tert-butyl ester to give 402 mg of 4-[(5-tert-butoxycarbonylamino-1-tert-butoxycarbonyl)pentylaminocarbonyl]-5-methyl-2-[(3-nitroimidazo[1,2-b]pyridazin-6-yl)thio]imidazole. This compound (211 mg) was dissolved in 5 ml of trifluoroacetic acid and the solution was allowed to stand at room temperature for 30 minutes. After evaporating trifluoroacetic acid, the residue was dissolved in 10 ml of acetonitrile, to which 0.105 ml of a concentrated hydrochloric acid was added. The solvent was evaporated to obtain crystals. The crystals were suspended in ethyl ether followed by filtration to give 187 mg of 4-[(5-amino-1-carboxy)pentylaminocarbonyl]-5-methyl-2-[(3-nitroimidazo[1,2-b]pyridazin-6-yl) thio]imidazole dihydrochloride, colorless crystals, m.p. 178-180°C.

| Elemental analysis for $C_{17}H_{20}N_8O_5S \cdot 2HCl$ | | | |
|---|---|---|---|
| Calculated: | C 39.16; | H 4.25; | N 21.49 |
| Found: | C 39.31; | H 4.19; | N 21.17 |

Example 15

Synthesis of 4-[(1,2-dicarboxy)ethylaminocarbonyl]-5-methyl-2-[(3-nitroimidazo[1,2-b]pyridazin-6-yl)thio]imidazole monohydrochloride.

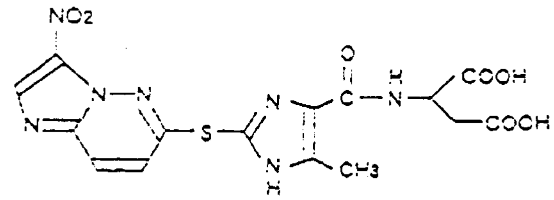

The same operations as in Examples 12 and 14 were carried out using 4-carboxy-5-methyl-2-mercaptoimidazole and di-tert-butyl aspartate to give 4-[(1,2-dicarboxy)ethylaminocarbonyl]-5-methyl-2-[(3-nitroimidazo[1,2-b]pyridazin-6-yl)thio)imidazole monohydrochloride, colorless crystals, m.p. 170-172°C.

| Elemental analysis for $C_{15}H_{13}N_7O_7S \cdot HCl$ | | | |
|---|---|---|---|
| Calculated: | C 38.17; | H 2.78; | N 20.78 |
| Found: | C 38.01; | H 2.54; | N 20.92 |

Preparation Example 1

Capsules

| | |
|---|---|
| (1) The compound prepared in Example 9-8 | 10 mg |
| (2) Lactose | 90 mg |
| (3) Microcrystalline cellulose | 70 mg |
| (4) Magnesium stearate | 10 mg |
| One capsule comprises: | 180 mg |

All amounts of the above-mentioned (1), (2) and (3) and 5 mg of (4) were mixed and granulated. Then the remaining amount (5 mg) of (4) was added thereto and the whole mixture was sealed in a gelatin capsule.

Preparation Example 2

Tablets

| | |
|---|---|
| (1) The compound prepared in Example 9-8 | 10 mg |
| (2) Lactose | 35 mg |
| (3) Corn starch | 150 mg |
| (4) Microcrystalline cellulose | 30 mg |
| (5) Magnesium stearate | 5 mg |
| One tablet comprises: | 230 mg |

All amounts of the above-mentioned (1), (2) and (3), 20 mg of (4) and 2.5 mg of (5) were mixed and granulated. Then the remaining 10 mg of (4) and 2.5 mg of (5) were added thereto followed by compressing to prepare a tablet.

Test Example 1

Antibacterial Activity Test against *Helicobacter pylori.*

Test Method

Antibacterial activity of each of the compounds which were synthesized in Examples 1 to 6, 9 and 10 was measured by the following method (an agar dilution method).

Each of the test compounds was dissolved in dimethyl sulfoxide and stepwisely diluted by a factor of 2 times with a sterilized distilled water whereby the test samples were prepared. Brucella agar to which 7% horse blood was added was used as a medium and 2 ml of each of the prepared test sample was mixed with 18 ml of the Brucella agar containing 7% of horse blood to prepare the plate for the measurement.

*Helicobacter pylori* (strains NCTC11637 and CPY433) was used as the microorganism for the test. Each of the strains was subjected to a shake culture at 37°C for 20 hours in a gas pack jar (to which CampyPak™ [BBL$^R$ Beckton Dickinson Microbiology Systems] was inserted) using a Brucella broth medium to which 2.5% bovine fetus serum was added.

Each five microliters of the microorganism liquids adjusted to a concentration of about $10^6$CFU/ml were inoculated on each of the plates for the measurement and cultured at 37°C for four days in a gas pack jar to which an absorbent cotton impregnated with water and CampyPak™ were inserted. After the culture, the growth of the microorganism was checked by naked eye and the lowest concentration where no growth of the strain of the microorganism was noted was taken as the MIC value (minimum growth inhibiting concentration) for each of the test compound.

Metronidazole (a known imidazole compound having an activity against *Helicobacter pylori*) was used as

a positive control and the MIC value for metronidazole was determined by the same method as in the case of the test compounds.

Result of the Tests

The result of the MIC values of the test compounds (the compounds synthesized in Examples 1 to 6, 9 and 10) and metronidazole (the positive control) is given in Table 4.

[Table 4]

| Antibacterial Activity against *Helicobacter pylori* | | |
|---|---|---|
| Compounds (Ex.Nos.) | MIC (micrograms/ml) | |
| | NCTC 11637 | CPY 433 |
| Example 1 | ≦0.05 | 0.78 |
| Example 2 | ≦0.05 | 0.39 |
| Example 3 | ≦0.05 | ≦0.05 |
| Example 4 | ≦0.05 | ≦0.05 |
| Example 5 | ≦0.05 | ≦0.05 |
| Example 6-1 | ≦0.05 | ≦0.05 |
| 6-3 | ≦0.006 | 0.013 |
| 6-4 | ≦0.006 | 0.013 |
| Example 9-1 | ≦0.006 | 0.025 |
| Example 10 | ≦0.006 | ≦0.006 |
| Metronidazole | 0.39 | 6.25 |

From the Table 4, it is apparent that, against all of the strains of *Helicobacter pylori* used in the test, the compounds of the present invention exhibit higher antibacterial activity than metronidazole.

The compound (I) of the present invention or its salt exhibits a high antibacterial activity against the microorganisms of the genus *Helicobacter* represented by *Helicobacter pylori*. Accordingly, when the compound (I) of the present invention or its salt is used, a favorable antibacterial effect is resulted with far less doses than the effective doses of the conventional antibacterial agents against the microorganisms belonging to the genus *Helicobacter*, especially against *Helicobacter pylori*.

The compound (I) of the present invention or its salt exhibits a high antibacterial activity especially against the microorganisms belonging to the genus *Helicobacter* represented by *Helicobacter pylori* and, therefore, it is effective as an antibacterial agent for preventing and/or treating various diseases caused by the microorganisms belonging to the genus *Helicobacter* such as duodenal ulcer, gastric ulcer, chronic gastritis, etc. Since *Helicobacter pylori* is also a big cause for the recurrence of ulcers, the compound (I) of the present invention or its salt is effective for preventing the recurrence of ulcers as well.

Further, the compound (I) of the present invention or its salt exhibits an excellent antibacterial activity against Gram-positive bacteria such as *Staphylococcus aureus* and also against Gram-negative bacteria such as *Hemophilus influenzae* and *Moraxella catarrhalis*. Consequently, the compound (I) of the present invention or its salt is effective as an antibacterial agent for preventing and/or treating various diseases caused by those microorganisms too.

In addition to the above-mentioned actions, the compound (I) of the present invention or its salt is effective as an antiulcer agent for preventing and/or treatnig the ulcers which are not caused by the microorganisms belonging to the genus *Helicobacter* such as stress ulcer.

The compound (I) of the present invention or its salt is stable and with low toxicity as well.

Thus, the present invention provides an excellent antibacterial and antiulcer agent having no adverse reactions.

## Claims

1. An imidazole compound of the formula (I'):

$$A'-(CH_2)_m-X-(CH_2)_n-B \qquad (I')$$

wherein A' is a condensed imidazolyl group having at least one nitro group on the imidazole ring, X is an oxygen atom or a sulfur atom, B is a 5- or 6-membered heterocyclic group which may be a condensed ring, and m and n are each an integer of 0 to 4, or its salt.

2. A compound according to Claim 1, in which B is a 5-membered heterocyclic group of the formula (VI):

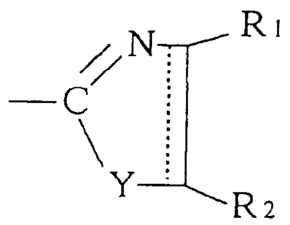

(VI)

wherein $R_1$ and $R_2$ are, the same or different, a hydrogen atom, a lower alkyl group which may be substituted, an amino group which may be substituted or a carboxyl group which may be substituted, or $R_1$ and $R_2$ together with carbon atoms to which they are attached may form an aromatic ring which may contain a nitrogen atom, and a bond between the two carbon atoms to which $R_1$ and $R_2$ are attached is a single bond or a double bond, and Y is an oxygen atom, a sulfur atom or a group of >$NR_4$ wherein $R_4$ is a hydrogen atom or an alkyl group which may be substituted.

3. A compound according to Claim 1, in which B is a 6-membered heterocyclic group of the formula (VII):

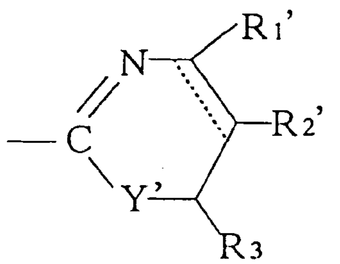

(VII)

wherein $R_1$' and $R_2$' are, the same or different, a hydrogen atom or a lower alkyl group, or $R_1$' and $R_2$' together with carbon atoms to which they are attached may form an aromatic ring which may contain a nitrogen atom, and a bond between the two carbon atoms to which $R_1$' and $R_2$' are attached is a single bond or a double bond, $R_3$ is a hydrogen atom, a hydrocarbon group or a heterocyclic group, and Y' is an oxygen atom, a sulfur atom or a group of >NH.

4. A compound according to Claim 1, in which the condensed imidazolyl group contains two to four nitrogen atoms as ring constituting atoms.

5. A compound according to Claim 1, in which A' is an imidazolyl group condensed with a 6-membered heterocycle.

6. A compound according to Claim 1, in which A' is an imidazolyl group condensed with a 6-membered heterocycle containing nitrogen atom(s).

7. A compound according to Claim 1, in which A' is an imidazolyl group condensed with pyridine, pyridazine, pyrimidine, pyrazine or triazine.

8. A compound according to Claim 1, in which A' is an imidazolyl group condensed with pyridine or pyridazine.

9. A compound according to Claim 1, in which A' is a 2- or 3-nitro-imidazo[1,2-b]pyridazin-6-yl group.

**10.** A compound according to Claim 1, in which A' is a 2- or 3-nitro-imidazo[1,2-a]pyridin-5-yl group.

**11.** A compound according to Claim 2, in which $R_1$ and $R_2$ are, the same or different, a hydrogen atom or a substituted lower alkyl group.

**12.** A compound according to Claim 2, in which either of $R_1$ and $R_2$ is a lower alkyl group substituted by a mono- or di-lower alkyl-amino group.

**13.** A compound according to Claim 2, in which $R_1$ and $R_2$ are, the same or different, a straight-chain or branched alkyl group of 1 to 6 carbon atoms.

**14.** A compound according to Claim 1, in which X is a sulfur atom.

**15.** A compound according to Claim 2, in which Y is a group of $>NR_4$ wherein $R_4$ is a hydrogen atom or a substituted lower alkyl group.

**16.** A compound according to Claim 1, in which both of m and n are 0.

**17.** A compound according to Claim 1, which is 6-[(imidazol -2-yl)thio]-3-nitroimidazo[1, 2-b]pyridazine, or its salt.

**18.** A compound according to Claim 1, which is 6-[[1-(2-hydroxyethyl)imidazol-2-yl]thio]-3-nitroimidazo[1,2-b]pyridazine, or its salt.

**19.** A compound according to Claim 1, which is 6-[(4-dimethylaminomethylimidazol-2-yl)thio]-3-nitroimidazo[1,2-b]pyridazine, or its salt.

**20.** A pharmaceutical composition which comprises a pharmacologically effective amount of an imidazole compound of the formula (I') as defined in Claim 1 or its pharmaceutically acceptable salt and a carrier or diluent.

**21.** Use of an imidazole compound of the formula (I):

$$A\text{-}(CH_2)_m\text{-}X\text{-}(CH_2)_n\text{-}B \qquad (I)$$

wherein A is an imidazol-1-yl or condensed imidazolyl group having at least one nitro group on the imidazole ring, X is an oxygen atom or a sulfur atom, B is a 5- or 6-membered heterocyclic group which may be a condensed ring, and m and n are each an integer of 0 to 4, as an active ingredient for the preparation of an antibacterial agent which comprises said compound or its pharmaceutically acceptable salt and a carrier or diluent.

**22.** Use according to Claim 21, in which A in the formula (I) is a condensed imidazolyl group having at least one nitro group on the imidazole ring.

**23.** Use according to Claim 21, in which the antibacterial agent is effective for a microorganism belonging to the genus *Helicobacter*.

**24.** Use according to Claim 21, in which the antibacterial agent is effective for *Helicobacter pylori*.

**25.** Use of an imidazole compound of the formula (I) as defined in claim 21 as an active ingredient for the preparation of an antiulcer agent which comprises said compound or its pharmaceutically acceptable salt and a carrier or diluent.

European Patent Office

# EUROPEAN SEARCH REPORT

Application Number
EP 94 30 4891

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,A | EP-A-0 535 528 (EURORESEARCH) 7 April 1993<br>* page 12, line 5 - page 14, line 9; claim 1 * | 1,21,23 | C07D487/04<br>A61K31/415<br>A61K31/50<br>C07D471/04<br>C07D519/00<br>A61K31/42<br>A61K31/425<br>A61K31/435<br>//(C07D487/04, 237:00, 235:00), (C07D471/04, 235:00, 221:00), (C07D519/00, 513:00,487:00) |
| A | WO-A-92 04898 (BYK GULDEN LOMBERG) 2 April 1992<br>* claims 1,10 * | 1,21,23 | |
| X | GB-A-1 388 915 (AMERICAN CYANAMID) 26 March 1975<br>* page 4, line 13 - line 25; claims 1,25; example 21 * | 1,20,21 | |
| X | GB-A-1 575 781 (MERCK) 24 September 1980<br>* example 10C * | 1 | |
| X | FARMACO,<br>vol.48, no.3, 1993, PAVIA IT<br>pages 443 - 446<br>S. DEMIRAYAK ET AL. 'Some nitroimidazole derivatives as possible antibacterial agents'<br>* tables I,II * | 21 | TECHNICAL FIELDS SEARCHED (Int.Cl.6) |
| X | ARZNEIMITTEL FORSCHUNG. DRUG RESEARCH,<br>vol.27, no.II, 1977, AULENDORF DE<br>pages 2251 - 2263<br>E. WINKELMANN ET AL. 'Chemotherapeutically active nitro compounds'<br>* tables 1.1,1.2,1.3 * | 21 | C07D<br>A61K |
| X | US-A-3 910 925 (SEARLE) 7 October 1975<br>* column 2, line 48 - column 3, line 36; claim 1 * | 21 | |
| X<br>D | FR-A-2 201 094 (SEARLE) 26 April 1974<br>* page 1, line 26 - line 31; claim 1 *<br>& GB-A-1 393 228 (SEARLE) 7 May 1975 | 21 | |
| | -/-- | | |

The present search report has been drawn up for all claims

| Place of search | Date of completion of the search | Examiner |
|---|---|---|
| THE HAGUE | 24 October 1994 | Alfaro Faus, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 30 4891

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | US-A-3 682 912 (R. PATRICK MAMALIS) 8 August 1972<br>* column 3, line 9 - line 18; claim 1 *<br>----- | 21 | |
| | | | TECHNICAL FIELDS SEARCHED (Int.Cl.6) |

The present search report has been drawn up for all claims

| Place of search | Date of completion of the search | Examiner |
|---|---|---|
| THE HAGUE | 24 October 1994 | Alfaro Faus, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)